# EUROPEAN PATENT APPLICATION

(11) **EP 3 991 777 A1**
(43) Date of publication of application: **04.05.2022**
(21) Application number: 19934720.4
(22) Date of filing: 11.10.2019
(51) Int. Cl.: A61M 25/10, A61M 29/00

(54) **BALLOON CATHETER**

(30) Priority: 28.06.2019 JP 2019120682
(71) Applicant: ASAHI INTECC CO., LTD., Seto-shi, Aichi 489-0071 (JP)
(72) Inventor: KATSURADA Takeharu, Seto-shi, Aichi 489-0071 (JP)
(74) Representative: Winter, Brandl - Partnerschaft mbB
(86) International application number: PCT/JP2019/040164
(87) International publication number: WO 2020/261591

(57) **Abstract**

A balloon catheter includes a tubular inner shaft, a balloon partly covering the inner shaft and having a distal end part joined to the inner shaft, and a tubular outer shaft accommodating a part of the inner shaft. The outer shaft includes a balloon joint part joined to a base end part of the balloon and a reduced diameter part being accommodated in the balloon and having a smaller outer diameter than the balloon joint part, and is joined to the inner shaft in the balloon. A communication hole communicating the expansion lumen and the inside of the balloon is formed. In the balloon, an outer diameter on the outermost periphery of a structure formed by the inner shaft, the X-ray opaque marker, and the outer shaft decreases continuously or stepwise from the base end part toward the distal end part of the balloon.

## Description

### TECHNICAL FIELD

The technique disclosed herein relates to a balloon catheter used to expand a constricted part or the like formed in a body cavity such as a blood vessel.

### BACKGROUND ART

A balloon catheter is used to expand a constricted part or an occluded part (hereinafter, referred to as a "lesion") formed in a body cavity such as a blood vessel. The balloon catheter includes a tubular inner shaft, a balloon that covers a part of the inner shaft and has a distal end part joined to the inner shaft, and a tubular outer shaft that accommodates a part of the inner shaft and is joined to a base end part of the balloon (see Patent Literature 1, for example). A distal end side of the balloon catheter is pushed into a lesion in a state where the balloon is contracted to follow outer shapes of the inner shaft and the outer shaft. Subsequently, a fluid for expanding the balloon is sent into the balloon from an expansion lumen formed between the outer shaft and the inner shaft, so that the balloon expands, and as a result, it is possible to widen and expand the lesion.

### CITATION LIST

### Patent Literature

Patent Literature 1: Japanese Unexamined Patent Application Publication No. 2002-291897
Patent Literature 2: US Patent Application Publication No. 2005/0273052
Patent Literature 3: Japanese Unexamined Patent Application Publication No. H10-33681
Patent Literature 4: US Patent No. 6315757
Patent Document 5: International Publication No. 2006/135581

### SUMMARY OF INVENTION

### TECHNICAL PROBLEM

In the conventional balloon catheter described above, a balloon joint part of the outer shaft to which a base end part of the balloon is joined is located at a distal end of the outer shaft and is arranged at a position separated radially outward from an outer peripheral surface of the inner shaft. Therefore, the balloon in the contracted state is contracted to follow a level difference in accordance with a difference in outer diameters between an outer peripheral surface of the balloon joint part of the outer shaft and the outer peripheral surface of the inner shaft. As a result, when the balloon is pushed into a narrow lesion, the distal end of the outer shaft bends, for example, due to the level difference, and the pushing force escapes to the side. Thus, the transmissibility of the pushing force from the outer shaft to the inner shaft is reduced, and as a result, there is a problem in that the passability of the balloon catheter decreases.

The present specification discloses a technique capable of solving the above-mentioned problems.

### MEANS FOR SOLVING PROBLEM

The techniques disclosed herein can be realized, for example, in the following modes.
(1) A balloon catheter disclosed herein is a balloon catheter including an inner shaft having a tubular shape, a balloon covering a part of the inner shaft and including a distal end part joined to the inner shaft, an X-ray opaque marker arranged on an inner peripheral surface side or an outer peripheral surface side of the inner shaft, and an outer shaft having a tubular shape and accommodating a part of the inner shaft, and in the balloon catheter, the outer shaft includes a balloon joint part joined to a base end part of the balloon and a reduced diameter part being accommodated in the balloon and having an outer diameter smaller than an outer diameter of the balloon joint part, the outer shaft being joined to the inner shaft in the balloon, the balloon catheter is formed with a communication hole communicating an inside of the balloon with an expansion lumen formed between an inner peripheral surface of the outer shaft and the outer peripheral surface of the inner shaft, and in the balloon, an outer diameter on an outermost periphery of a structure formed by the inner shaft, the X-ray opaque marker, and the outer shaft decreases continuously or stepwise from the base end part toward the distal end part of the balloon.
   In the present balloon catheter, a fluid for expanding the balloon can be sent from the expansion lumen into the balloon, via the communication hole communicating the expansion lumen and the balloon. Further, the outer shaft includes the reduced diameter part, and the reduced diameter part is accommodated in the balloon and has an outer diameter smaller than an outer diameter of the balloon joint part joined to the base end part of the balloon. As a result, the balloon in a contracted state decreases in diameter to follow an outer shape of the reduced diameter part of the outer shaft from the base end part joined to the balloon joint part of the outer shaft toward the distal end part. Thus, in the present balloon catheter, as compared, for example, with a configuration in which the outer shaft does not include the reduced diameter part, it is possible to suppress a decrease in the transmissibility of a pushing force from the outer shaft to the inner shaft due to a difference in outer diameters between the balloon joint part of the outer shaft and the inner shaft in the balloon. Further, in the present balloon catheter, the outer shaft is joined to the inner shaft in the balloon. Therefore, compared with a configuration in which the outer shaft is not joined to the inner shaft in the balloon, the pushing force from the outer shaft to the inner shaft is efficiently transmitted. Thereby, according to the present balloon catheter, it is possible to improve the passability of the balloon catheter. Further, in the present balloon catheter, in the balloon, the outer diameter on the outermost periphery of the structure formed by the inner shaft, the X-ray opaque marker, and the outer shaft decreases continuously or stepwise from the base end part toward the distal end part of the balloon. Therefore, the passability of the balloon catheter when moving the balloon catheter to the distal end side after the balloon catheter reaches a lesion is further improved, and it is possible to suppress damage to the balloon due to the balloon getting stuck when the balloon catheter is moved to the base end side.
(2) The balloon catheter described above may have a configuration in which a distal end of the reduced diameter part in the outer shaft is joined to the inner shaft. In the present balloon catheter, the distal end of the reduced diameter part in the outer shaft is joined to the inner shaft. Therefore, compared with, for example, a configuration in which a portion of the outer shaft, which is nearer the base end than the reduced diameter part is, is joined to the inner shaft, the pushing force from the outer shaft to the inner shaft is transmitted even more efficiently. Thus, according to the present balloon catheter, it is possible to improve the passability of the balloon catheter more effectively.
(3) The balloon catheter described above may have a configuration in which a thickness of a distal end part of the outer shaft is thinner than a thickness of the balloon joint part of the outer shaft. In the present balloon catheter, a rigidity of the distal end part of the outer shaft is lower than a rigidity of the balloon joint part of the outer shaft, and thus, it is possible to suppress, for example, the occurrence of a kink in which the distal end part of the outer shaft is bent and does not return to its original state.
(4) The balloon catheter described above may have a configuration in which a thickness of the outer shaft decreases continuously or stepwise from the balloon joint part of the outer shaft toward the distal end part of the outer shaft. In the present balloon catheter, the rigidity of the outer shaft decreases continuously or stepwise from the balloon joint part of the outer shaft toward the distal end part, and thus, it is possible to effectively suppress the occurrence of a kink in the outer shaft, for example.
(5) The balloon catheter described above may have a configuration in which the X-ray opaque marker is arranged on a distal end side of the outer shaft in an axial direction of the inner shaft, and a distance between a base end of the X-ray opaque marker and the distal end of the outer shaft in the axial direction is less than twice a thickness of the balloon. According to the present balloon catheter, compared with a configuration in which the X-ray opaque marker is not arranged at the distal end side of the inner shaft, a position of a distal end part of the balloon catheter in a living body can be imaged more accurately. Further, according to the present balloon catheter, compared with a configuration in which the distance between the base end of the X-ray opaque marker and the distal end of the outer shaft in the axial direction of the inner shaft is equal to or more than twice the thickness of the balloon, it is possible to suppress a decrease in an expansion function of the balloon and damage to the balloon due to, for example, the balloon entering a space between the base end of the X-ray opaque marker and the distal end of the outer shaft.
(6) The balloon catheter described above may have a configuration in which the X-ray opaque marker is arranged on the distal end side of the outer shaft in the axial direction of the inner shaft, and an outer diameter of the X-ray opaque marker is equal to or smaller than an outer diameter of the distal end of the reduced diameter part. In the present balloon catheter, compared with a configuration in which the outer diameter of the X-ray opaque marker is larger than the outer diameter of the distal end of the reduced diameter part, it is possible to suppress a decrease in the transmissibility of the pushing force from the outer shaft to the inner shaft due to the difference in outer diameters between the X-ray opaque marker and the inner shaft, and the passability of the balloon catheter can be further improved.
(7) The balloon catheter described above may have a configuration in which the X-ray opaque marker is arranged nearer a base end than a joint portion between the inner shaft and the outer shaft is. In the present balloon catheter, the balloon and the X-ray opaque marker do not contact each other, and thus, it is possible to suppress damage to the balloon (for example, a rupture of the balloon) caused by a contact between the balloon and the X-ray opaque marker.
(8) The balloon catheter described above may have a configuration in which the X-ray opaque marker is arranged at a position corresponding to the balloon joint part of the outer shaft or nearer a distal end than the position corresponding to the balloon joint part of the outer shaft is, in the axial direction of the inner shaft. According to the present balloon catheter, compared with a configuration in which the X-ray opaque marker is arranged nearer the base end than the position corresponding to the balloon joint part of the outer shaft is in the axial direction of the inner shaft, it is possible to accurately image a position of the distal end part of the balloon catheter in the living body, while damage to the balloon caused by a contact between the balloon and the X-ray opaque marker can be suppressed.
(9) The balloon catheter described above may have a configuration in which the outer diameter of the balloon joint part of the outer shaft is smaller than an outer diameter of a portion of the outer shaft nearer a base end than the balloon joint part is. In the present balloon catheter, compared with a configuration in which the outer diameter of the balloon joint part of the outer shaft is equal to or greater than the outer diameter of the portion of the outer shaft nearer the base end than the balloon joint part is, the passability of the balloon catheter when moving the balloon catheter to the distal end side after the balloon catheter reaches a lesion is improved, and it is possible to suppress damage to the balloon due to the balloon getting stuck when the balloon catheter is moved to the base end side.
(10) The balloon catheter described above may have a configuration in which an outer peripheral surface of the reduced diameter part of the outer shaft decreases continuously in diameter from the balloon joint part toward the distal end part of the outer shaft. In the present balloon catheter, the outer peripheral surface of the reduced diameter part decreases continuously in diameter from the balloon joint part toward the distal end part of the outer shaft. Therefore, according to the present balloon catheter, compared with, for example, a configuration in which the entire outer peripheral surface of the reduced diameter part is parallel to the axial direction of the outer shaft, it is possible to more effectively suppress a decrease in the transmissibility of the pushing force from the outer shaft to the inner shaft due to the difference in outer diameters between the balloon joint part of the outer shaft and the inner shaft and the passability of the balloon catheter can be further improved.
(11) The balloon catheter described above may have a configuration in which the outer peripheral surface of the reduced diameter part of the outer shaft decreases in diameter in a plurality of steps from the balloon joint part toward the distal end part of the outer shaft. In the present balloon catheter, the outer peripheral surface of the reduced diameter part decreases in diameter in a plurality of steps from the balloon joint part toward the distal end part of the outer shaft. Therefore, according to the present balloon catheter, compared with, for example, a configuration in which the entire outer peripheral surface of the reduced diameter part is parallel to the axial direction of the outer shaft, it is possible to more effectively suppress a decrease in the transmissibility of the pushing force from the outer shaft to the inner shaft due to the difference in outer diameters between the balloon joint part of the outer shaft and the inner shaft and the passability of the balloon catheter can be further improved.
(12) The balloon catheter described above may have a configuration in which the reduced diameter part includes a separated portion separated from the outer peripheral surface of the inner shaft in a radially outward direction of the inner shaft, and the communication hole is formed in the separated portion. Assuming a configuration in which the communication hole communicating the expansion lumen and the inside of the balloon is formed between the inner peripheral surface of the outer shaft and the outer peripheral surface of the inner shaft (at a joint portion between both shafts in the balloon), the outer diameter of the outer shaft is increased for securing the diameter of the communication hole, and as a result, the pushability of the balloon catheter may decrease. On the other hand, in the present balloon catheter, the communication hole is formed in the separated portion of the reduced diameter part of the outer shaft, separated from the inner shaft. Therefore, it is possible to suppress an increase in the diameter of the outer shaft due to the formation of the communication hole.
(13) The balloon catheter described above may have a configuration in which the distal end of the reduced diameter part of the outer shaft is arranged at a position separated from a joint portion between the inner shaft and the balloon in the axial direction of the inner shaft. In the present balloon catheter, the distal end of the reduced diameter part of the outer shaft is arranged at a position separated from the joint portion between the inner shaft and the balloon. Thus, according to the present balloon catheter, compared with, for example, a configuration in which the distal end of the reduced diameter part of the outer shaft extends to the joint portion between the inner shaft and the balloon, the diameter of a distal end side portion of the balloon in the contracted state is small, and thus, it is possible to improve the pushability of the distal end side portion of the balloon catheter into the lesion in the contracted state of the balloon.
(14) The balloon catheter described above may have a configuration in which the reduced diameter part of the outer shaft is integrally formed with a portion of the outer shaft adjacent to the reduced diameter part. According to the present balloon catheter, compared with a configuration in which the reduced diameter part is formed separately from a portion of the outer shaft adjacent to the reduced diameter part, it is possible to suppress a decrease in the passability of the balloon catheter due to a difference in rigidity between the reduced diameter part and the portion adjacent to the reduced diameter part.
(15) The balloon catheter described above may have a configuration in which the reduced diameter part of the outer shaft is formed by extending the distal end of the outer shaft. According to the present balloon catheter, when the rigidity of the outer shaft decreases continuously from the balloon joint part toward the distal end part of the outer shaft, it is possible to more effectively suppress a decrease in the passability of the balloon catheter due to a difference in rigidity between the reduced diameter part and the portion adjacent to the reduced diameter part.

### BRIEF DESCRIPTION OF DRAWINGS

FIG. 1 is an explanatory view (longitudinal sectional view) schematically illustrating a configuration of a balloon catheter 100 according to a first embodiment.
FIG. 2 is an explanatory view (transverse sectional view) schematically illustrating a configuration of the balloon catheter 100 according to the first embodiment.
FIGs. 3A to 3D are an explanatory view illustrating a usage example of the balloon catheter 100 according to the first embodiment.
FIGs. 4A and 4B are an explanatory view (longitudinal sectional view) schematically illustrating a contracted state of a balloon 30 in the balloon catheter 100 of the first embodiment and in a balloon catheter 100X of a comparative example.
FIG. 5 is an explanatory view (longitudinal sectional view) schematically illustrating a configuration of a balloon catheter 100a according to a second embodiment.
FIG. 6 is an explanatory view (transverse sectional view) schematically illustrating a configuration of the balloon catheter 100a according to the second embodiment.
FIG. 7 is an explanatory view (longitudinal sectional view) schematically illustrating a configuration of a balloon catheter 100b according to a first modification.
FIG. 8 is an explanatory view (longitudinal sectional view) schematically illustrating a configuration of a balloon catheter 100c according to a second modification.
FIG. 9 is an explanatory view (longitudinal sectional view) schematically illustrating a configuration of a balloon catheter 100d according to a third modification.
FIG. 10 is an explanatory view (longitudinal sectional view) schematically illustrating a configuration of a balloon catheter 100e according to a fourth modification.
FIG. 11 is an explanatory view (longitudinal sectional view) schematically illustrating a configuration of a balloon catheter 100f according to a fifth modification.
FIG. 12 is an explanatory view (longitudinal sectional view) schematically illustrating a configuration of a balloon catheter 100g according to a sixth modification.
FIG. 13 is an explanatory view (longitudinal sectional view) schematically illustrating a configuration of a balloon catheter 100h according to a seventh modification.

### EMBODIMENTS OF THE INVENTION

### A. First Embodiment:

### A-1. Basic Configuration of Balloon Catheter 100:

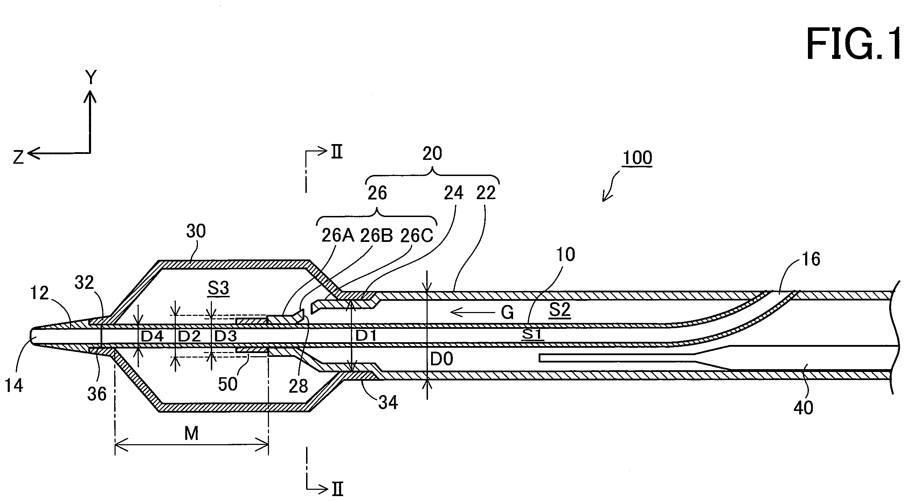

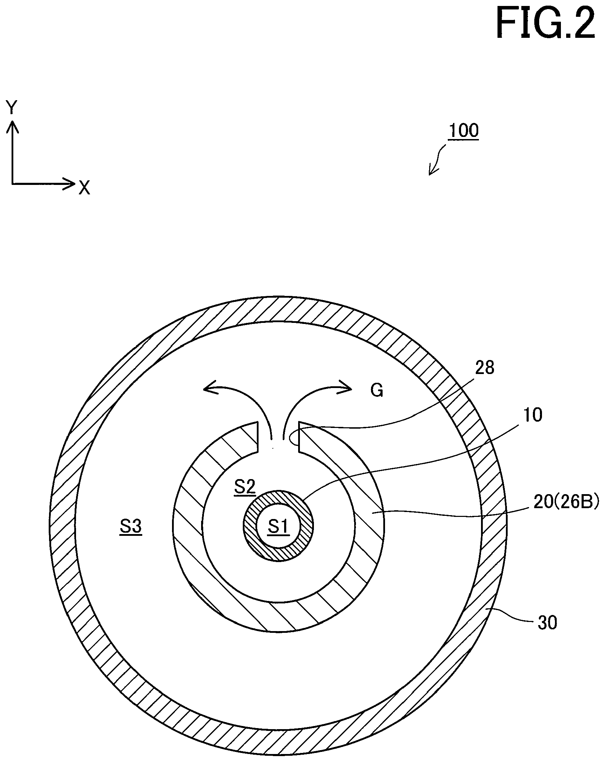

FIGS. 1 and 2 are explanatory views schematically illustrating a configuration of a balloon catheter 100 according to a first embodiment. FIG. 1 illustrates a configuration of a lateral cross-section of the balloon catheter 100 (a YZ-cross section: a cross-sectional view cut along a plane including a Y-axis and a Z-axis illustrated in FIG. 1). FIG. 2 illustrates a configuration of a cross section of the balloon catheter 100 at a position II-II in FIG. 1 (an XY-cross section: a cross-sectional view cut along a plane including an X-axis and a Y-axis illustrated in FIG. 2). In FIG. 1, a positive direction side of the Z-axis (a distal tip 12 of the balloon catheter 100) is a distal end side (distal side) inserted into the body, and a negative direction side of the Z-axis (a side opposite to the distal tip 12 of the balloon catheter 100) is a base end side (proximal side) operated by a qualified person such as a doctor. It is noted that FIG. 1 illustrates a state where the balloon catheter 100 has a general linear shape parallel to the Z-axis direction, however, the balloon catheter 100 is sufficiently soft to be bent. Further, FIGS. 1 and 2 illustrate a state where a balloon 30 described later is expanded.

The balloon catheter 100 is a medical device to be inserted into a blood vessel or the like to widen and expand a lesion (a constricted part or an occluded part) in the blood vessel or the like. The balloon catheter 100 includes an inner shaft 10, an outer shaft 20, and the balloon 30.

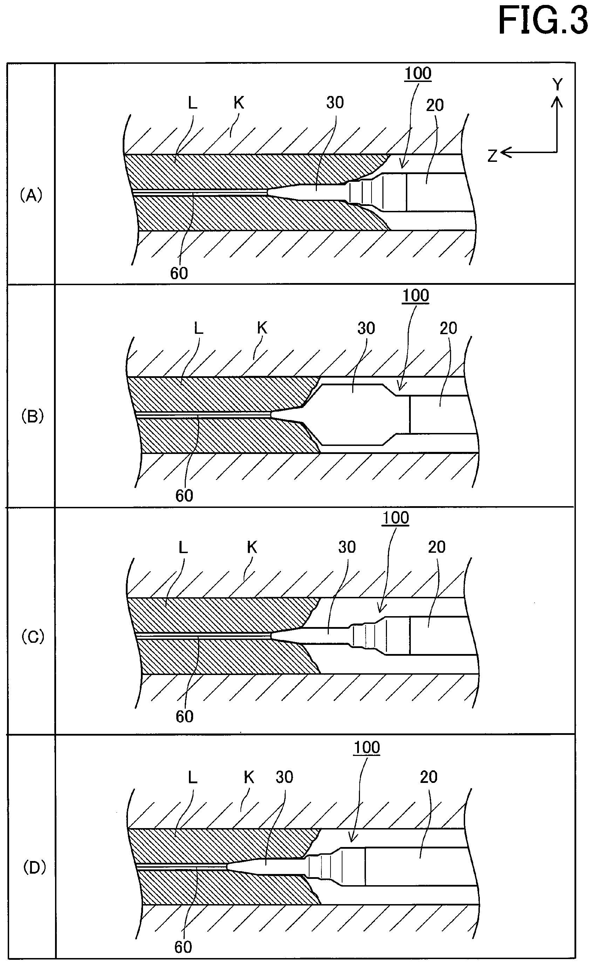

The inner shaft 10 is a tubular (for example, a hollow cylindrical) member having a distal end and a base end that are open. It is noted that the meaning of "tubular (hollow cylindrical)" herein is not limited to a perfectly tubular shape (hollow cylindrical shape), and the shape may be substantially tubular in general (for example, a substantially hollow cylindrical shape such as a slightly conical shape and a shape partially having an unevenness). A guide wire lumen S1 through which a guide wire 60 (see FIGs. 3A to 3D described later) is inserted is formed inside the inner shaft 10. It is noted that the distal tip 12 is provided at the distal end of the inner shaft 10. The distal tip 12 is a tubular member having a distal end and a rear end that are open. The distal tip 12 has a tapered outer shape in which a distal end side guide wire port 14 is formed on a distal end side of the distal tip 12 and the outer diameter gradually decreases toward the distal end. The guide wire 60 inserted into the guide wire lumen S1 is led to the outside from the distal end side guide wire port 14 (see FIGs. 3A to 3D described later). It is noted that the distal tip 12 is formed of a resin.

The outer shaft 20 is a tubular (for example, a hollow cylindrical) member having a distal end and a base end that are open. An inner diameter of the outer shaft 20 is larger than an outer diameter of the inner shaft 10. The outer shaft 20 accommodates a part of the inner shaft 10 and is arranged to be located coaxially with the inner shaft 10. An expansion lumen S2 through which a fluid G for expansion used for expanding the balloon 30 flows, is formed between an outer peripheral surface of the inner shaft 10 and an inner peripheral surface of the outer shaft 20. It is noted that the fluid G may be a gas or a liquid, and examples thereof include gases such as helium gas, CO₂ gas, and O₂ gas, and liquids such as physiological saline and a contrast medium.

Specifically, the outer shaft 20 includes a shaft main body part 22 having a tubular shape with an annular cross section and a balloon joint part 24 being located on a distal end side of the outer shaft 20 with respect to the shaft main body part 22 and having an inner diameter and an outer diameter that are smaller than those of the shaft main body part 22. Inner peripheral surfaces of the shaft main body part 22 and the balloon joint part 24 are separated from the outer peripheral surface of the inner shaft 10 over the entire circumference around the axis (Z-axis direction) of the inner shaft 10 (see FIG. 1). It is noted that, in FIG. 1, a portion of the shaft main body part 22 is not illustrated.

The distal end part of the inner shaft 10 protrudes toward the distal end side from the distal end part of the outer shaft 20. The base end of the inner shaft 10 is curved to the side with respect to an axial direction of the inner shaft 10 (a length direction of the inner shaft 10, that is, the Z-axis direction in each figure) to be connected to a side wall of the shaft main body part 22 of the outer shaft 20, and is open on the outer peripheral surface of the shaft main body part 22. The opening on the outer peripheral surface of the shaft main body part 22 forms a base end side guide wire port 16 of the inner shaft 10, and the guide wire 60 is inserted from the base end side guide wire port 16. That is, the balloon catheter 100 in the present embodiment is a so-called rapid exchange type catheter.

The inner shaft 10 and the outer shaft 20 are formed of a material having a heat-sealability and a certain degree of flexibility. Examples of the material for forming the inner shaft 10 and the outer shaft 20 include thermoplastic resins, more specifically, polyolefins such as polyethylene, polypropylene, polybutene, ethylene-propylene copolymers, ethylene-vinyl acetate copolymers, ionomers, or mixtures of two or more types of these compounds, polyvinyl chloride resin, polyamides, polyamide elastomers, polyesters, polyester elastomers, and thermoplastic polyurethane.

It is noted that, as illustrated in FIG. 1, a core wire 40 is accommodated inside the shaft main body part 22 of the outer shaft 20. The core wire 40 is a rod-shaped member having a small diameter at a distal end side and a large diameter at a base end side. The core wire 40 applies a rigidity change to the balloon catheter 100 so that the balloon catheter 100 is softer toward the distal end. For example, the core wire 40 is formed of a metal material, more specifically, stainless steel (SUS302, SUS304, SUS316, and the like), a superelastic alloy such as an Ni-Ti alloy, a piano wire, a nickel-chromium based alloy, a cobalt alloy, tungsten, and the like.

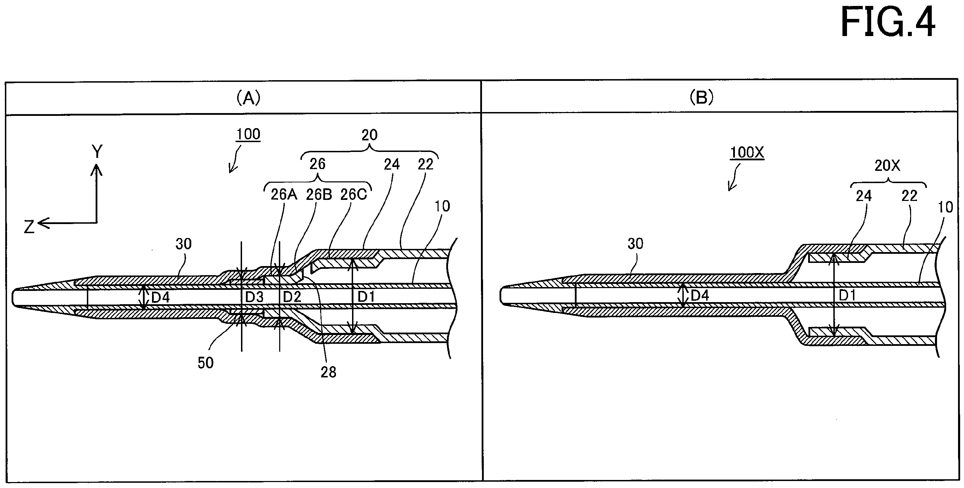

The balloon 30 is an expansion part expandable and contractible in accordance with the supply and discharge of the fluid G. The balloon 30 covers a distal end part of the inner shaft 10 protruding from the distal end of the outer shaft 20. Further, a distal end part 32 of the balloon 30 is joined to the inner shaft 10 (the outer peripheral surface on the base end side of the distal tip 12) by welding, for example, and a base end part 34 of the balloon 30 is joined to the outer peripheral surface of the balloon joint part 24 in the outer shaft 20 by welding, for example. The distal end part of the distal tip 12 is open on a more distal end side than the distal end part 32 of the balloon 30. It is noted that, in the contracted state, the balloon 30 is folded to be in close contact with the outer peripheral surfaces of the inner shaft 10 and the outer shaft 20 (see FIGS. 3 and 4A described later).

The balloon 30 is preferably formed of a material having a certain degree of flexibility, and is more preferably formed of a material being thinner than the inner shaft 10 and the outer shaft 20 and having flexibility. Examples of the material for forming the balloon 30 include polyolefins such as polyethylene, polypropylene, polybutene, ethylene-propylene copolymers, ethylene-vinyl acetate copolymers, ionomers, or mixtures of two or more types of these, thermoplastic resins such as soft polyvinyl chloride resin, polyamides, polyamide elastomers, polyesters, polyester elastomers, polyurethane, and fluororesin, silicone rubber, and latex rubber.

### A-2. Detailed Configuration of Balloon Catheter 100:

Next, a detailed configuration of the balloon catheter 100 of the present embodiment will be described. As illustrated in FIGS. 1 and 2, in the balloon catheter 100 of the present embodiment, the outer shaft 20 includes a reduced diameter part 26. The reduced diameter part 26 is located on the distal end side with respect to the balloon joint part 24. That is, the reduced diameter part 26 is accommodated in an internal space S3 of the balloon 30. The reduced diameter part 26 has a tubular shape (for example, a tubular shape having an annular cross section). An outer diameter of the reduced diameter part 26 is smaller than an outer diameter D1 of the balloon joint part 24. Specifically, the reduced diameter part 26 includes a small-diameter part 26A, a taper part 26B, and a tubular part 26C. As illustrated in FIG. 1, the reduced diameter part 26 may include a portion having a tubular shape (for example, the small-diameter part 26A or the tubular part 26C). The shape of the small-diameter part 26A is a tubular shape (for example, a hollow cylindrical shape) having an outer peripheral surface parallel to the outer peripheral surface of the inner shaft 10, and an outer diameter D2 of the small-diameter part 26A is larger than an outer diameter D4 of the inner shaft 10 and smaller than the outer diameter D1 of the balloon joint part 24. The taper part 26B is located between the small-diameter part 26A and the balloon joint part 24 in the axial direction (Z-axis direction) of the inner shaft 10. A diameter of an outer peripheral surface of the taper part 26B decreases continuously from a boundary position between the taper part 26B and the balloon joint part 24 toward a boundary position between the taper part 26B and the small-diameter part 26A. It is noted that the diameter of the outer peripheral surface of the taper part 26B may decrease linearly toward the distal end side, or may decrease in a curved shape toward the distal end side. It is noted that the balloon joint part 24 refers to a portion of the outer shaft 20 that is joined to the balloon 30. Thus, as illustrated in FIG. 1, the balloon joint part 24 does not include a portion of the outer shaft 20 (for example, the tubular part 26C) that is not in contact with (joined to) the balloon 30 in an expanded state of the balloon 30.

As illustrated in FIG. 1, the inner peripheral surface of the small-diameter part 26A in the outer shaft 20 is joined to the outer peripheral surface of the inner shaft 10 by welding, for example. The small-diameter part 26A is an example of a distal end of a reduced diameter part in CLAIMS.

Further, as illustrated in FIG. 1, an inner peripheral surface of the taper part 26B in the outer shaft 20 is separated from the outer peripheral surface of the inner shaft 10 outward in the radial direction of the inner shaft 10, and a communication hole 28 is formed to penetrate the taper part 26B. As illustrated in FIG. 2, the communication hole 28 is formed at one position in the taper part 26B in a circumferential direction around an axis of the balloon catheter 100. The expansion lumen S2 formed between the outer peripheral surface of the inner shaft 10 and the inner peripheral surface of the outer shaft 20 communicates with the internal space S3 of the balloon 30 via the communication hole 28. Therefore, it is possible to supply and discharge the fluid G between the expansion lumen S2 and the internal space S3 of the balloon 30. The taper part 26B is an example of a separated portion in CLAIMS.

Further, as illustrated in FIG. 1, the distal end of the reduced diameter part 26 (the small-diameter part 26A) of the outer shaft 20 is arranged at a position separated from a joint portion 36 between the inner shaft 10 and the distal end part 32 of the balloon 30 by a distance M, for example, in the axial direction (Z-axis direction) of the inner shaft 10.

Moreover, the balloon catheter 100 of the present embodiment includes an X-ray opaque marker 50. The X-ray opaque marker 50 is a tubular (for example, a hollow cylindrical) member, is located at the distal end side of the outer shaft 20 with respect to the reduced diameter part 26, and is arranged to surround the outer peripheral surface of the inner shaft 10. The X-ray opaque marker 50 is formed of a metal such as gold, platinum, and tungsten. Thus, when the balloon catheter 100 is inserted into a living body, the position of the X-ray opaque marker 50 can be imaged by X-rays from the outside of the living body. It is noted that a base end of the X-ray opaque marker 50 is adjacent to the distal end of the reduced diameter part 26 of the outer shaft 20. Further, as illustrated in FIG. 1, an outer diameter D3 of the X-ray opaque marker 50 is larger than the outer diameter D4 of the inner shaft 10 and smaller than the outer diameter D2 of the small-diameter part 26A of the outer shaft 20.

Relational Expression 1 below is satisfied for the outer diameters of the inner shaft 10, the outer shaft 20, and the X-ray opaque marker 50, and thus, as described above, the outer diameter gradually decreases from the balloon joint part 24 toward the outer peripheral surface of the inner shaft 10.

### Relational Expression 1

Outer diameter D1 of balloon joint part 24 > outer diameter of taper part 26B > outer diameter D2 of small-diameter part 26A > outer diameter D3 of X-ray opaque marker 50 > outer diameter D4 of inner shaft 10

Therefore, in a state where the fluid G is not sent into the internal space S3 and the balloon 30 is contracted (hereinafter, simply referred to as a "contracted state"), the diameter of the balloon 30 gradually decreases to follow the outer shapes of the reduced diameter part 26 of the outer shaft 20, the inner shaft 10, and the X-ray opaque marker 50, from the base end part 34 joined to the balloon joint part 24 of the outer shaft 20 toward the distal end part 32. That is, the outer diameter of the balloon 30 also gradually decreases from the base end part 34 toward the distal end part 32 (see FIG. 4A described later).

It is noted that, for example, an outer diameter D0 of the outer shaft 20 is 0.77 mm, the outer diameter D1 of the balloon joint part 24 in the outer shaft 20 is 0.75 mm, the outer diameter D2 of the small-diameter part 26A in the reduced diameter part 26 is 0.57 mm, the outer diameter D3 of the X-ray opaque marker 50 is 0.54 mm, and the outer diameter D4 of the inner shaft 10 is 0.48 mm. Further, the difference between the outer diameter D1 of the balloon joint part 24 and the outer diameter D2 of the small-diameter part 26A is 0.18 mm, the difference between the outer diameter D2 of the small-diameter part 26A and the outer diameter D3 of the X-ray opaque marker 50 is 0.03 mm, and the difference between the outer diameter D3 of the X-ray opaque marker 50 and the outer diameter D4 of the inner shaft 10 is 0.06 mm.

### A-3. Usage Example of Balloon Catheter 100:

Next, a usage example of the balloon catheter 100 in the first embodiment will be described. FIGs. 3A to 3D are an explanatory view illustrating a usage example of the balloon catheter 100 in the first embodiment. First, the guide wire 60 is inserted into a blood vessel K. The rear end of the guide wire 60 is inserted into the distal end side guide wire port 14 of the balloon catheter 100, and the balloon catheter 100 is inserted into the blood vessel K along the guide wire 60. Next, the balloon catheter 100 is pushed toward the distal end side in a state where the balloon 30 is contracted, to guide the balloon catheter 100 along the guide wire 60 to a lesion L in the blood vessel K. Here, as described above, the balloon 30 in the contracted state is in close contact with the outer peripheral surfaces of the inner shaft 10 and the outer shaft 20, and the outer diameter of the balloon 30 gradually decreases from the base end part 34 to the distal end part 32. Therefore, if the outer shaft 20 is pushed toward the distal end side, as illustrated in FIG. 3A, a balloon distal end portion of the balloon catheter 100 from the distal tip 12 to a portion in the balloon 30 covering the inner shaft 10 and the reduced diameter part 26 of the outer shaft 20 can be relatively easily inserted into the lesion L.

Next, in a state where the balloon distal end portion in the balloon catheter 100 is inserted into the lesion L, the fluid G is sent from the expansion lumen S2 into the internal space S3 of the balloon 30 to expand the balloon 30. Therefore, as illustrated in FIG. 3B, the lesion L is widened by the expanded balloon 30. Subsequently, as illustrated in FIG. 3C, the balloon 30 in the balloon catheter 100 is again returned from the expanded state to the contracted state, and as illustrated in FIG. 3D, the balloon catheter 100 is pushed to the distal end side in the contracted state of the balloon 30, to insert the balloon distal end portion further to a back side of the lesion L. If this procedure is repeated, it is possible to widen the lesion L while advancing the balloon 30 toward the back side of the lesion L.

### A-4. Effects of Present Embodiment

As described above, in the balloon catheter 100 of the present embodiment, the fluid G for expanding the balloon 30 can be sent from the expansion lumen S2 into the internal space S3 of the balloon 30, via the communication hole 28 formed in the outer shaft 20. Further, the outer shaft 20 includes the reduced diameter part 26, and the reduced diameter part 26 is accommodated in the balloon 30 and has an outer diameter smaller than the outer diameter D1 of the balloon joint part 24 joined to the base end part 34 of the balloon 30. As a result, the diameter of the balloon 30 in the contracted state gradually decreases to follow the outer shape of the reduced diameter part 26 of the outer shaft 20 from the base end part 34 joined to the balloon joint part 24 of the outer shaft 20 toward the distal end part 32.

Here, FIGs. 4A and 4B are an explanatory view schematically illustrating the contracted state of the balloon 30 in the balloon catheter 100 of the present embodiment and in a balloon catheter 100X of a comparative example. FIG. 4A illustrates a state where the balloon 30 is contracted in the balloon catheter 100 of the present embodiment, and FIG. 4B illustrates a state where the balloon 30 is contracted in the balloon catheter 100X of the comparative example. As illustrated in FIG. 4B, the balloon catheter 100X of the comparative example is different from the balloon catheter 100 of the present embodiment in that the balloon catheter 100X does not include the reduced diameter part 26. Therefore, the diameter of the balloon 30 in the contracted state decreases to follow a relatively large level difference in accordance with a difference in outer diameters (D1 and D4) between the outer peripheral surface of the balloon joint part 24 and the outer peripheral surface of the inner shaft 10. As a result, when the balloon 30 is pushed into a narrow lesion, the distal end of the outer shaft 20 bends, for example, and the pushing force escapes to the side due to this large level difference. Thus, the transmissibility of the pushing force from the outer shaft 20 to the inner shaft 10 is reduced, and as a result, there is a problem in that the passability of the balloon catheter 100 decreases.

On the other hand, as illustrated in FIG. 4A, in the balloon catheter 100 of the present embodiment, the outer shaft 20 includes the reduced diameter part 26. Therefore, the diameter of the balloon 30 in the contracted state gradually decreases to follow the outer shape of the reduced diameter part 26 of the outer shaft 20 from the base end part 34 joined to the balloon joint part 24 of the outer shaft 20 toward the distal end part 32. Thus, in the present embodiment, as compared, for example, with the comparative example in which the outer shaft 20 does not include the reduced diameter part 26, it is possible to suppress a decrease in the transmissibility of the pushing force from the outer shaft 20 to the inner shaft 10 due to the difference in outer diameters between the balloon joint part 24 of the outer shaft 20 and the inner shaft 10 in the balloon 30. Further, in the present embodiment, the outer shaft 20 is joined to the inner shaft 10 in the balloon 30. Therefore, compared with a configuration in which the outer shaft 20 is not joined to the inner shaft 10 in the balloon 30, the pushing force from the outer shaft 20 to the inner shaft 10 is efficiently transmitted. Thereby, according to the present embodiment, it is possible to improve the passability of the balloon catheter 100. Further, in the present embodiment, in the balloon 30, the outer diameter D0 on the outermost periphery of a structure formed by the inner shaft 10, the X-ray opaque marker 50, and the outer shaft 20 decreases continuously or stepwise from the base end part toward the distal end part of the balloon 30. That is, from the base end part toward the distal end part of the balloon 30, there is no large level difference that may damage the balloon. Therefore, the passability of the balloon catheter 100 when moving the balloon catheter 100 to the distal end side after the balloon catheter 100 reaches the lesion is further improved, and it is also possible to suppress damage to the balloon 30 due to the balloon 30 getting stuck when the balloon catheter 100 is moved to the base end side.

Further, in the present embodiment, the distal end of the reduced diameter part 26 in the outer shaft 20 is joined to the inner shaft 10. Therefore, for example, compared with a configuration in which a portion of the outer shaft 20, which is nearer the base end than the reduced diameter part 26 is, is joined to the inner shaft 10, the pushing force from the outer shaft 20 to the inner shaft 10 is transmitted even more efficiently. Further, it is possible to suppress a deformation in the shape of the reduced diameter part 26 due to a load from the pushing force. Thereby, according to the present embodiment, it is possible to improve the passability of the balloon catheter 100 more effectively.

Further, in the present embodiment, the diameter of the outer peripheral surface of the reduced diameter part 26 decreases in a plurality of steps from the balloon joint part 24 toward the distal end part of the outer shaft 20. Therefore, according to the present embodiment, compared with, for example, a configuration in which the entire outer peripheral surface of the reduced diameter part 26 is parallel to the axial direction (the Z-axis direction) of the outer shaft 20, it is possible to more effectively suppress a decrease in the transmissibility of the pushing force from the outer shaft 20 to the inner shaft 10 due to the difference in outer diameters between the balloon joint part 24 of the outer shaft 20 and the inner shaft 10 and the passability of the balloon catheter 100 can be further improved.

Assuming a configuration in which the communication hole 28 communicating the expansion lumen S2 and the internal space S3 of the balloon 30 is formed between the inner peripheral surface of the outer shaft 20 and the outer peripheral surface of the inner shaft 10, the outer diameter of the outer shaft 20 is increased for securing the diameter of the communication hole 28, and as a result, the pushability of the balloon catheter 100 may decrease. On the other hand, in the present embodiment, the communication hole 28 is formed in the taper part 26B of the reduced diameter part 26 of the outer shaft 20 which is separated from the inner shaft 10. Therefore, it is possible to suppress an increase in the diameter of the outer shaft 20 due to the formation of the communication hole 28.

Further, in the present embodiment, the distal end of the reduced diameter part 26 of the outer shaft 20 is arranged at a position separated from the joint portion 36 of the inner shaft 10 and the balloon 30. Thus, according to the present embodiment, compared with, for example, a configuration in which the distal end of the reduced diameter part 26 of the outer shaft 20 extends to the joint portion 36 of the inner shaft 10 and the balloon 30, the diameter of a distal end side portion of the balloon 30 in the contracted state is small, and thus, it is possible to improve the pushability of the distal end side portion of the balloon catheter 100 into the lesion in the contracted state of the balloon 30.

In the present embodiment, the X-ray opaque marker 50 is arranged at the distal end side of the outer shaft 20 in the axial direction of the inner shaft 10. Here, the distance between the base end of the X-ray opaque marker 50 and the distal end of the outer shaft 20 in the axial direction is preferably less than twice the thickness of the balloon 30. Thus, compared with a configuration in which the X-ray opaque marker 50 is not arranged at the distal end side of the inner shaft 10, a position of the distal end part of the balloon catheter 100 in the living body can be imaged accurately. Further, compared with a configuration in which the distance between the base end of the X-ray opaque marker 50 and the distal end of the outer shaft 20 in the axial direction is equal to or more than twice the thickness of the balloon 30, it is possible to suppress a decrease in an expansion function of the balloon 30 and damage to the balloon 30 due to, for example, the balloon 30 entering a space between the base end of the X-ray opaque marker 50 and the distal end of the outer shaft 20.

Further, in the present embodiment, the X-ray opaque marker 50 having an outer diameter smaller than the outer diameter of the reduced diameter part 26 of the outer shaft 20 is provided. Therefore, according to the present embodiment, compared with, for example, a configuration in which the outer diameter of the X-ray opaque marker 50 located at the distal end side of the reduced diameter part 26 is larger than the outer diameter of the reduced diameter part 26, it is possible to suppress a decrease in the transmissibility of the pushing force from the outer shaft 20 to the inner shaft 10 due to the difference in outer diameters between the X-ray opaque marker 50 and the inner shaft 10, and the passability of the balloon catheter 100 can be further improved.

Further, in the present embodiment, the outer diameter D1 of the balloon joint part 24 of the outer shaft 20 is smaller than the outer diameter D0 of a portion (the shaft main body part 22) of the outer shaft 20 nearer the base end than the balloon joint part 24 is. According to the present embodiment, compared with, for example, a configuration in which the outer diameter of the balloon joint part 24 of the outer shaft 20 is equal to or greater than the outer diameter of the shaft main body part 22, the passability of the balloon catheter 100 when moving the balloon catheter 100 to the distal end side after the balloon catheter 100 reaches the lesion is improved, and it is possible to suppress damage to the balloon 30 due to the balloon 30 getting stuck when the balloon catheter 100 is moved to the base end side.

Further, in the present embodiment, the reduced diameter part 26 of the outer shaft 20 is formed integrally with the shaft main body part 22 of the outer shaft 20. According to the present embodiment, compared with a configuration in which the reduced diameter part 26 and the shaft main body part 22 are formed separately, it is possible to suppress a decrease in the passability of the balloon catheter 100 due to a difference in rigidity between the reduced diameter part 26 and the shaft main body part 22.

### B. Second Embodiment

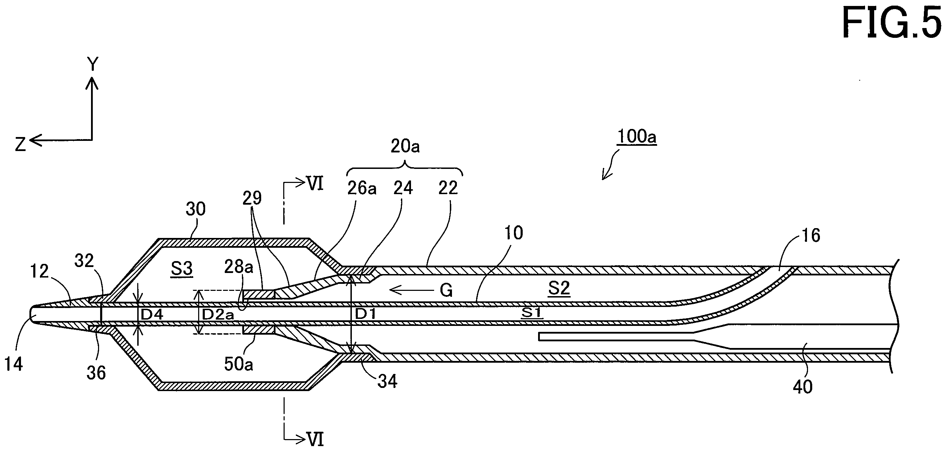

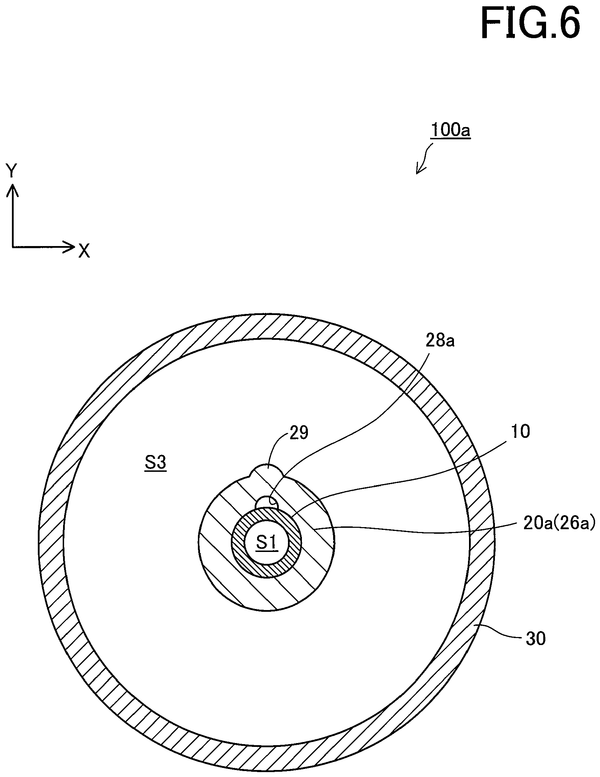

FIGS. 5 and 6 are explanatory views schematically illustrating a configuration of a balloon catheter 100a according to a second embodiment. FIG. 5 illustrates a configuration of a lateral cross section (a YZ-cross section) of the balloon catheter 100a, and FIG. 6 illustrates a configuration of a cross section (an XY-cross section) of the balloon catheter 100a at a position indicated by VI-VI in FIG. 5. In the following, elements in the configuration of the balloon catheter 100a of the second embodiment that are the same as those of the balloon catheter 100 of the first embodiment described above will be referred to by the same reference numerals and description thereof will be omitted where appropriate.

As illustrated in FIG. 5, in the balloon catheter 100a of the second embodiment, a configuration of a reduced diameter part 26a in an outer shaft 20a is different from the balloon catheter 100 of the first embodiment. That is, in the balloon catheter 100a of the second embodiment, a diameter of an outer peripheral surface of the reduced diameter part 26a decreases continuously from a boundary position between the reduced diameter part 26a and the balloon joint part 24 toward a distal end of the reduced diameter part 26a (a boundary position between the reduced diameter part 26a and an X-ray opaque marker 50a). The distal end of the reduced diameter part 26a is adjacent to a base end of the X-ray opaque marker 50a and an outer diameter of the distal end of the reduced diameter part 26a is the same as an outer diameter D2a of the base end of the X-ray opaque marker 50a, and thus, there is no level difference between the reduced diameter part 26a and the X-ray opaque marker 50a. Further, the outer diameter of the base end of the reduced diameter part 26a is the same as the outer diameter D1 of the distal end of the balloon joint part 24, and thus, there is no level difference between the reduced diameter part 26a and the balloon joint part 24. It is noted that the diameter of the outer peripheral surface of the reduced diameter part 26a may decrease linearly toward the distal end side, or may decrease in a curved shape toward the distal end side. It is noted that "M and N are the same" herein does not mean that M and N are exactly the same, and a difference between M and N may be a value equal to or less than 1% of M.

Further, in the balloon catheter 100a of the second embodiment, a communication hole 28a is formed between inner peripheral surfaces of the reduced diameter part 26a in the outer shaft 20a and the X-ray opaque marker 50a, and the outer peripheral surface of the inner shaft 10. Specifically, as illustrated in FIGS. 5 and 6, a groove extending along the axial direction (the Z-axis direction) of the inner shaft 10 is formed on the inner peripheral surfaces of the reduced diameter part 26a and the X-ray opaque marker 50a, and a space surrounded by the groove and the outer peripheral surface of the inner shaft 10 forms the communication hole 28a. It is noted that, in the present embodiment, to secure a strength (a thickness) of a groove-forming portion in the reduced diameter part 26a and the X-ray opaque marker 50a, a protruding portion 29 protruding outward in the radial direction is formed in a portion of the outer peripheral surfaces of the reduced diameter part 26a and the X-ray opaque marker 50a corresponding to the groove.

As described above, in the balloon catheter 100a of the second embodiment, the diameter of the outer peripheral surface of the reduced diameter part 26a decreases continuously from the balloon joint part 24 toward the distal end part of the outer shaft 20a. That is, in the present embodiment, there is no level difference from the balloon joint part 24 to the distal end part of the outer shaft 20a. Therefore, according to the present embodiment, compared with, for example, a configuration in which the entire outer peripheral surface of the reduced diameter part 26a is parallel to the axial direction (the Z-axis direction) of the inner shaft 10 and a configuration in which the diameter of the reduced diameter part 26a decreases in a plurality of steps, it is possible to more effectively suppress a decrease in the transmissibility of the pushing force from the outer shaft 20a to the inner shaft 10 due to the difference in outer diameters between the balloon joint part 24 of the outer shaft 20a and the inner shaft 10 and the passability of the balloon catheter 100a can be further improved.

### C. Modifications:

The technique disclosed herein is not limited to the above-described embodiment, and may be modified into various modes without departing from the spirit of the above-described embodiment. For example, the following modifications can be applied.

The configuration of the balloon catheter 100 in the embodiment described above is merely an example and various modifications can be applied. For example, in the above-described embodiment, the outer diameter of the balloon joint part 24 in the outer shaft 20 may be equal to or larger than the outer diameter of the shaft main body part 22. Further, the above-described first embodiment may have a configuration in which the communication hole 28 is formed at a plurality of positions in the taper part 26B in the circumferential direction around the axis of the balloon catheter 100.

In the above-described embodiment, the distal end of the reduced diameter part 26 (the small-diameter part 26A) in the outer shaft 20 is configured to be joined to the inner shaft 10. However, the base end of the reduced diameter part 26 (for example, a portion in which the thickness of a part of the taper part 26B is increased) may be configured to be joined to the inner shaft 10, for example. That is, it is only required that the outer shaft 20 is configured to be joined to the inner shaft 10 in the balloon 30. Further, in the above-described embodiment, the distal end of the reduced diameter part 26 of the outer shaft 20 may be configured to extend to the joint portion 36 of the inner shaft 10 and the balloon 30.

In the first embodiment described above, instead of the taper part 26B, the reduced diameter part 26 may be configured to include a stepped part having a stepped surface perpendicular to the axial direction of the outer shaft 20. It is noted that, "M and N are perpendicular" herein does not mean that an angle formed by M and N is limited to an angle of 90 degrees, and it is only required that the angle formed by M and N is equal to or less than 90 degrees ± 5 degrees. In this case, the number of steps in the entire reduced diameter part 26 may be one step or a plurality of steps. Further, in the first embodiment described above, the communication hole 28 may be configured to be formed between the inner peripheral surface of the outer shaft 20 and the outer peripheral surface of the inner shaft 10. Moreover, in the second embodiment described above, the outer shaft 20a may have a configuration in which a communication hole is formed to penetrate the reduced diameter part 26a.

In the first embodiment described above, the outer diameter D3 of the X-ray opaque marker 50 may be equal to the outer diameter D2 of the small-diameter part 26A in the outer shaft 20. Further, in the second embodiment described above, the outer diameter of the base end of the X-ray opaque marker 50a may be smaller or larger than the outer diameter of the distal end of the reduced diameter part 26a. Moreover, in the second embodiment described above, a configuration may be employed in which a level difference is provided between the base end of the reduced diameter part 26a and the distal end of the balloon joint part 24. Further, in each of the above-described embodiments, the base ends of the X-ray opaque markers 50 and 50a may be separated from the distal ends of the reduced diameter parts 26 and 26a of the outer shaft 20. Moreover, in each of the above-described embodiments, a configuration may be employed in which a plurality of the X-ray opaque markers 50 and 50a are provided, or the X-ray opaque markers 50 and 50a are not provided.

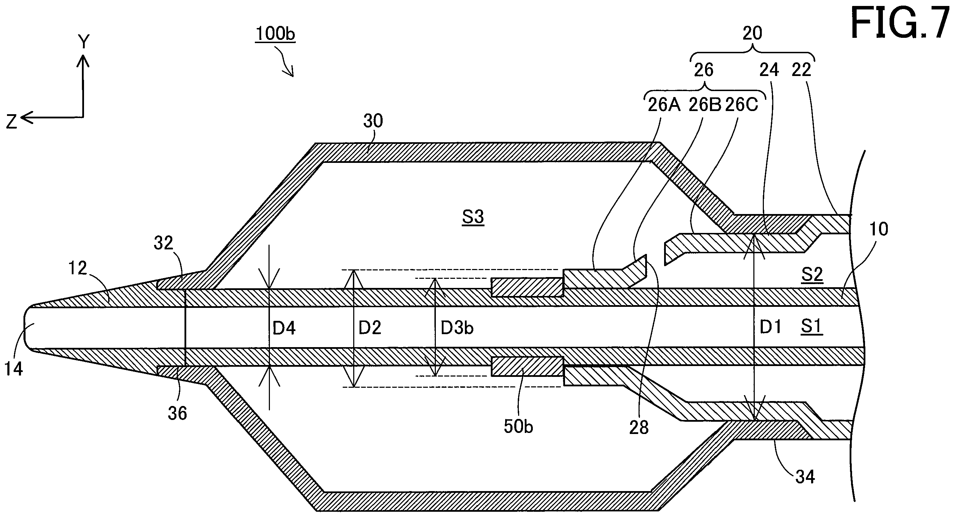

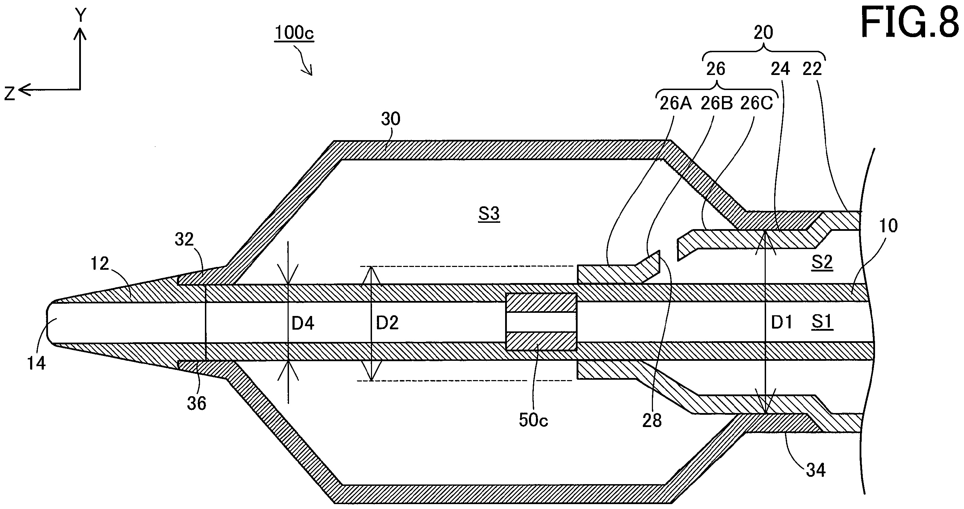

FIG. 7 is an explanatory view schematically illustrating a configuration of a balloon catheter 100b according to a first modification. FIG. 8 is an explanatory view schematically illustrating a configuration of a balloon catheter 100c according to a second modification. Elements in the configurations of the balloon catheters 100b and 100c of each of the first and second modifications that are the same as those of the balloon catheter 100 of the first embodiment described above will be referred to by the same reference numerals and description thereof will be omitted where appropriate. In the balloon catheters 100b and 100c of each of the first and second modifications, an arrangement of X-ray opaque markers 50b and 50c is different from that in the balloon catheter 100 of the first embodiment. That is, as illustrated in FIG. 7, in the balloon catheter 100b in the first modification, an inner peripheral surface side of the X-ray opaque marker 50b is embedded in the inner shaft 10. With such a configuration, it is possible to reduce a level difference between an outer peripheral surface of the X-ray opaque marker 50b and the outer peripheral surface of the inner shaft 10. It is noted that, in the first modification, the X-ray opaque marker 50b may be entirely embedded in the inner shaft 10 and the outer peripheral surface of the X-ray opaque marker 50b and the outer peripheral surface of the inner shaft 10 may be continuously connected without a level difference. That is, the outer peripheral surface of the X-ray opaque marker 50b and the outer peripheral surface of the inner shaft 10 may be arranged on the same plane or curved plane without forming a level difference. As illustrated in FIG. 8, in the balloon catheter 100c in the second modification, the X-ray opaque marker 50c is arranged on the inner peripheral surface side of the inner shaft 10 and an outer peripheral surface side of the X-ray opaque marker 50c is embedded in the inner shaft 10. With such a configuration, it is possible to suppress the occurrence of a level difference on the outer peripheral surface of the balloon catheter 100c due to the presence of the X-ray opaque marker 50c. It is noted that, in the second modification, the X-ray opaque marker 50c may be entirely embedded in the inner shaft 10 and an inner peripheral surface of the X-ray opaque marker 50c may be flush with the inner peripheral surface of the inner shaft 10. That is, the inner peripheral surface of the X-ray opaque marker 50c and the inner peripheral surface of the inner shaft 10 may be arranged on the same plane or curved plane without forming a level difference.

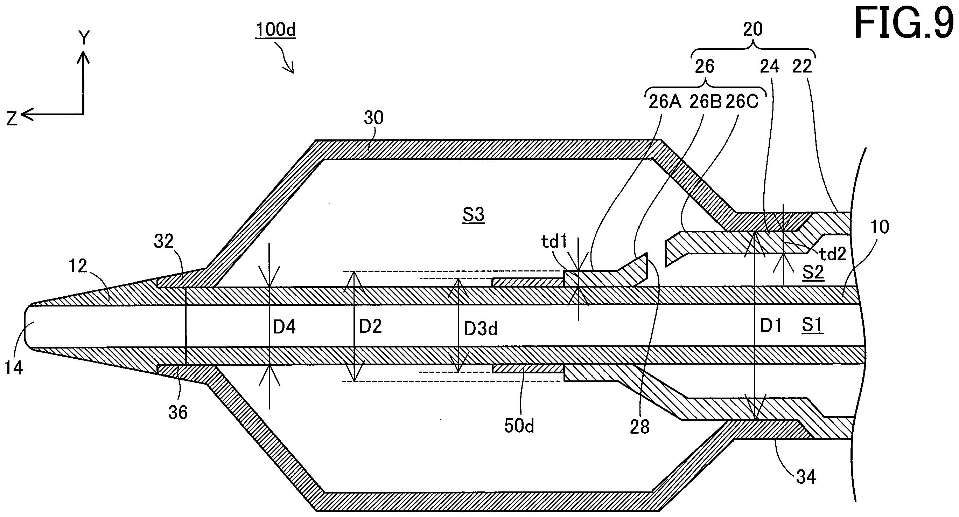

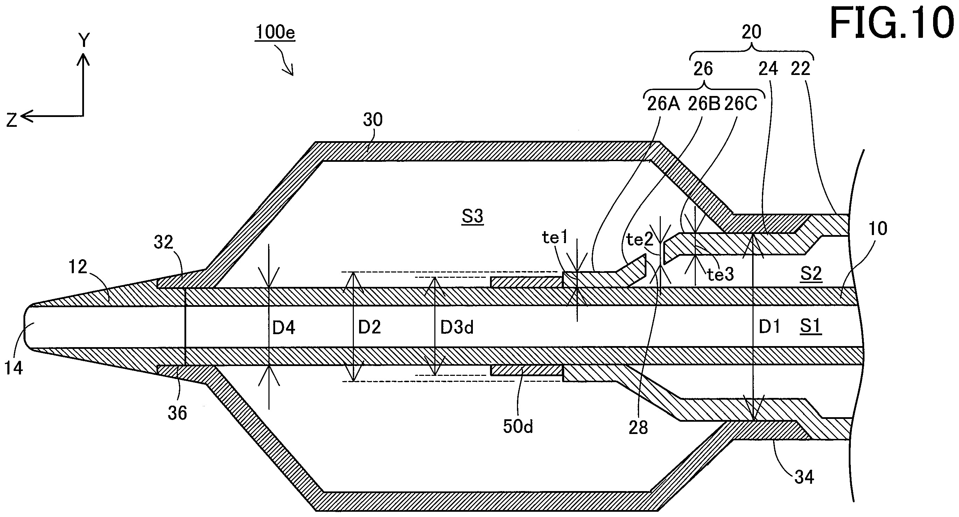

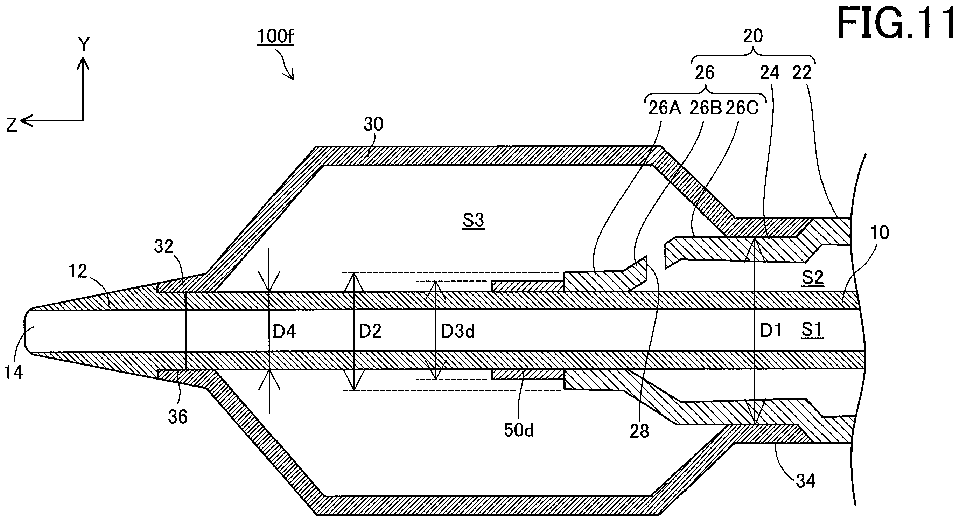

FIG. 9 is an explanatory view schematically illustrating a configuration of a balloon catheter 100d according to a third modification. FIG. 10 is an explanatory view schematically illustrating a configuration of a balloon catheter 100e according to a fourth modification. FIG. 11 is an explanatory view schematically illustrating a configuration of a balloon catheter 100f according to a fifth modification. Elements in the configurations of the balloon catheters 100d to 100f of each of the third to fifth modifications that are the same as those of the balloon catheter 100b of the first modification described above will be referred to by the same reference numerals and description thereof will be omitted where appropriate. As illustrated in FIG. 9, the balloon catheter 100d of the third modification is different from the balloon catheter 100b of the first modification in that a thickness (a distance between the inner peripheral surface and the outer peripheral surface of the outer shaft 20 in the radial direction, the same applying hereinafter) td1 of the distal end part (the small-diameter part 26A) of the outer shaft 20 is thinner than a thickness td2 of the balloon joint part 24 of the outer shaft 20. Therefore, in the third modification, the rigidity of the distal end part of the outer shaft 20 is lower than the rigidity of the balloon joint part 24 of the outer shaft 20, and thus, it is possible to suppress, for example, the occurrence of a kink in which the distal end part of the outer shaft 20 is bent and does not return to its original state. Further, in the third modification, an X-ray opaque marker 50d is arranged to surround the outer peripheral surface of the inner shaft 10. Moreover, Relational Expression 2 below is satisfied for the outer diameters of the inner shaft 10, the outer shaft 20, and the X-ray opaque marker 50d, and thus, the outer diameter gradually decreases from the balloon joint part 24 toward the outer peripheral surface of the inner shaft 10.

### Relational Expression 2

Outer diameter D1 of balloon joint part 24 > outer diameter D2 of small-diameter part 26A > outer diameter D3d of X-ray opaque marker 50d > outer diameter D4 of inner shaft 10

Further, as illustrated in FIG. 10, the balloon catheter 100e of the fourth modification is different from the balloon catheter 100b of the first modification in that the thickness of the outer shaft 20 decreases stepwise from the balloon joint part 24 of the outer shaft 20 toward the distal end part of the outer shaft 20. Specifically, Relational Expression 3 below is satisfied for the thicknesses of the small-diameter part 26A, the taper part 26B, and the tubular part 26C (the balloon joint part 24).

### Relational Expression 3

Thickness te1 of small-diameter part 26A < thickness te2 of taper part 26B < thickness te3 of tubular part 26C (balloon joint part 24)

Therefore, in the fourth modification, the rigidity of the outer shaft 20 decreases stepwise from the balloon joint part 24 toward the distal end part of the outer shaft 20, and thus, it is possible to effectively suppress the occurrence of a kink in the outer shaft 20, for example. It is noted that, in the fourth modification, similarly to the third modification described above, the X-ray opaque marker 50d is arranged to surround the outer peripheral surface of the inner shaft 10.

Further, as illustrated in FIG. 11, the balloon catheter 100f of the fifth modification is different from the balloon catheter 100b of the first modification in that the thickness of the outer shaft 20 decreases continuously from the balloon joint part 24 of the outer shaft 20 toward the distal end part of the outer shaft 20. Specifically, the reduced diameter part 26 of the outer shaft 20 is formed by extending the distal end side of the outer shaft 20. Therefore, according to the fifth modification, when the rigidity of the outer shaft 20 decreases continuously from the balloon joint part 24 toward the distal end part of the outer shaft 20, it is possible to more effectively suppress a decrease in the passability of the balloon catheter 100f due to a difference in rigidity between the reduced diameter part 26 and a portion adjacent to the reduced diameter part 26 (for example, the shaft main body part 22). It is noted that, in the fifth modification, similarly to the third modification described above, the X-ray opaque marker 50d is arranged to surround the outer peripheral surface of the inner shaft 10.

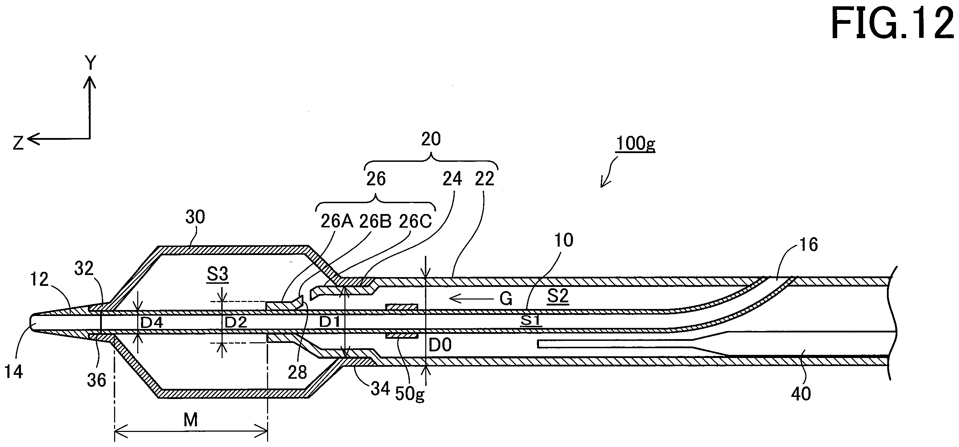

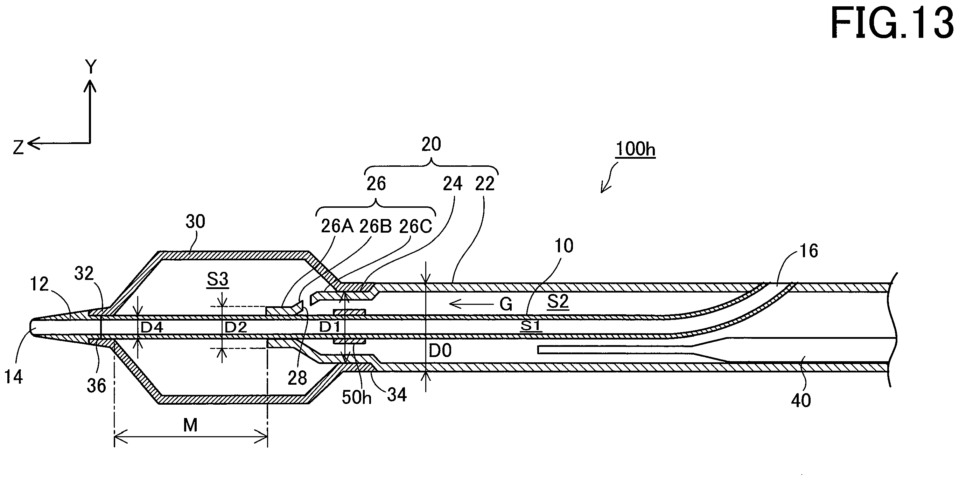

FIG. 12 is an explanatory view schematically illustrating a configuration of a balloon catheter 100g according to a sixth modification. FIG. 13 is an explanatory view schematically illustrating a configuration of a balloon catheter 100h according to a seventh modification. Elements in the configurations of the balloon catheters 100g and 100h of each of the sixth and seventh modifications that are the same as those of the balloon catheter 100 of the first embodiment described above will be referred to by the same reference numerals and description thereof will be omitted where appropriate. As illustrated in FIG. 12, the balloon catheter 100g of the sixth modification is different from the balloon catheter 100 of the first embodiment in that an X-ray opaque marker 50g is arranged nearer a base end than a joint portion between the inner shaft 10 and the outer shaft 20 (in FIG. 12, a contact portion between the inner peripheral surface of the small-diameter part 26A and the outer peripheral surface of the inner shaft 10) is. In the sixth modification, the balloon 30 and the X-ray opaque marker 50g do not contact each other, and thus, it is possible to suppress damage to the balloon 30 (for example, a rupture of the balloon 30) caused by a contact between the balloon 30 and the X-ray opaque marker 50g.

As illustrated in FIG. 13, the balloon catheter 100h of the seventh modification is different from the balloon catheter 100 of the first embodiment in that an X-ray opaque marker 50h is arranged at a position corresponding to the balloon joint part 24 of the outer shaft 20 in the axial direction. It is noted that the X-ray opaque marker 50h may be arranged nearer the distal end than the position corresponding to the balloon joint part 24 is. In the seventh modification, compared with a configuration in which the X-ray opaque marker 50h is arranged nearer the base end than the position corresponding to the balloon joint part 24 of the outer shaft 20 is in the axial direction, it is possible to accurately image a position of the distal end part of the balloon catheter 100h in the living body, while damage to the balloon 30 caused by a contact between the balloon 30 and the X-ray opaque marker 50h can be suppressed.

Further, the material of each member in the above-described embodiment is merely an example and various modifications can be applied.

In the embodiment described above, a configuration in which the present invention is applied to the rapid exchange type balloon catheter 100 is described in an example, but the present invention may be applied to a so-called over-the-wire type balloon catheter.

### DESCRIPTION OF REFERENCE NUMERALS

10: Inner shaft
12: Distal tip
14: Distal end side guide wire port
16: Base end side guide wire port
20: Outer shaft
20a: Outer shaft
22: Shaft main body part
24: Balloon joint part
26, 26a: Reduced diameter part
26A: Small-diameter part
26B: Taper part
28, 28a: Communication hole
29: Protruding portion
30: Balloon
32: Distal end part
34: Base end part
36: Joint portion
40: Core wire
50, 50a - 50h: X-ray opaque marker
60: Guide wire
100, 100X, 100 a - 100h: Balloon catheter
G: Fluid
K: Blood vessel
L: Lesion
S1: Guide wire lumen
S2: Expansion lumen
S3: Internal space

## Claims

1. A balloon catheter, comprising:
an inner shaft having a tubular shape;
a balloon covering a part of the inner shaft and including a distal end part joined to the inner shaft;
an X-ray opaque marker arranged on an inner peripheral surface side or an outer peripheral surface side of the inner shaft; and
an outer shaft having a tubular shape and accommodating a part of the inner shaft, wherein
the outer shaft includes a balloon joint part joined to a base end part of the balloon and a reduced diameter part being accommodated in the balloon and having an outer diameter smaller than an outer diameter of the balloon joint part, the outer shaft being joined to the inner shaft in the balloon,
the balloon catheter is formed with a communication hole communicating an inside of the balloon with an expansion lumen formed between an inner peripheral surface of the outer shaft and the outer peripheral surface of the inner shaft, and
in the balloon, an outer diameter on an outermost periphery of a structure formed by the inner shaft, the X-ray opaque marker, and the outer shaft decreases continuously or stepwise from the base end part toward the distal end part of the balloon.

2. The balloon catheter according to claim 1, wherein
a distal end of the reduced diameter part in the outer shaft is joined to the inner shaft.

3. The balloon catheter according to claim 1 or 2, wherein
a thickness of a distal end part of the outer shaft is thinner than a thickness of the balloon joint part of the outer shaft.

4. The balloon catheter according to claim 3, wherein
a thickness of the outer shaft decreases continuously or stepwise from the balloon joint part of the outer shaft toward the distal end part of the outer shaft.

5. The balloon catheter according to any one of claims 1 to 4, wherein
the X-ray opaque marker is arranged on a distal end side of the outer shaft in an axial direction of the inner shaft, and a distance between a base end of the X-ray opaque marker and the distal end of the outer shaft in the axial direction is less than twice a thickness of the balloon.

6. The balloon catheter according to any one of claims 1 to 5, wherein
the X-ray opaque marker is arranged on the distal end side of the outer shaft in the axial direction of the inner shaft, and an outer diameter of the X-ray opaque marker is equal to or smaller than an outer diameter of the distal end of the reduced diameter part.

7. The balloon catheter according to any one of claims 1 to 4, wherein
the X-ray opaque marker is arranged nearer a base end than a joint portion between the inner shaft and the outer shaft is.

8. The balloon catheter according to claim 7, wherein
the X-ray opaque marker is arranged at a position corresponding to the balloon joint part of the outer shaft or nearer a distal end than the position corresponding to the balloon joint part of the outer shaft is, in an axial direction of the inner shaft.

9. The balloon catheter according to any one of claims 1 to 8, wherein
the outer diameter of the balloon joint part of the outer shaft is smaller than an outer diameter of a portion of the outer shaft nearer a base end than the balloon joint part is.

10. The balloon catheter according to any one of claims 1 to 9, wherein
an outer peripheral surface of the reduced diameter part of the outer shaft decreases continuously in diameter from the balloon joint part toward the distal end part of the outer shaft.

11. The balloon catheter according to any one of claims 1 to 10, wherein
the outer peripheral surface of the reduced diameter part of the outer shaft decreases in diameter in a plurality of steps from the balloon joint part toward the distal end part of the outer shaft.

12. The balloon catheter according to any one of claims 1 to 11, wherein
the reduced diameter part includes a separated portion separated from the outer peripheral surface of the inner shaft in a radially outward direction of the inner shaft, and the communication hole is formed in the separated portion.

13. The balloon catheter according to any one of claims 1 to 12, wherein
the distal end of the reduced diameter part of the outer shaft is arranged at a position separated from a joint portion between the inner shaft and the balloon in the axial direction of the inner shaft.

14. The balloon catheter according to any one of claims 1 to 13, wherein
the reduced diameter part of the outer shaft is integrally formed with a portion of the outer shaft adjacent to the reduced diameter part.

15. The balloon catheter according to claim 14, wherein
the reduced diameter part of the outer shaft is formed by extending the distal end of the outer shaft.
